**Europäisches Patentamt**

**European Patent Office**

**Office européen des brevets**

(11) Veröffentlichungsnummer: **0 294 613 B1**

# (12) EUROPÄISCHE PATENTSCHRIFT

(45) Veröffentlichungstag der Patentschrift:
27.03.91 Patentblatt 91/13

(51) Int. Cl.⁵ : **C07C 311/00**

(21) Anmeldenummer: **88107713.5**

(22) Anmeldetag: **13.05.88**

(54) Verfahren und Anlage zur kontinuierlichen Herstellung von Chlorsulfonylisocyanat.

(30) Priorität: **10.06.87 DE 3719303**

(43) Veröffentlichungstag der Anmeldung:
**14.12.88 Patentblatt 88/50**

(45) Bekanntmachung des Hinweises auf die
Patenterteilung:
**27.03.91 Patentblatt 91/13**

(84) Benannte Vertragsstaaten:
**AT BE CH DE ES FR GB IT LI NL**

(56) Entgegenhaltungen:
**DE-B- 1 159 410**
**DE-B- 1 171 887**
**DE-C- 928 896**

(73) Patentinhaber: **HOECHST
AKTIENGESELLSCHAFT
Postfach 80 03 20
W-6230 Frankfurt am Main 80 (DE)**

(72) Erfinder: **Niermann, Hermann, Dr.
Am Wachberg 34
W-5042 Erftstadt (DE)**
Erfinder: **Diskowski, Herbert, Dr.
Fasanenaue 4
W-5042 Erftstadt (DE)**
Erfinder: **Roszinski, Hilmar, Dr.
Julius-Leber-Strasse 7
W-5042 Erftstadt (DE)**
Erfinder: **Tiedemann, Jens, Dr.
Graf-Stauffenberg-Strasse 4
W-5042 Erftstadt (DE)**
Erfinder: **Martin, Willi
Von-Stephan-Strasse 42
W-5042 Erftstadt (DE)**

## Beschreibung

Die vorliegende Erfindung betrifft ein Verfahren zur kontinuierlichen Herstellung von Chlorsulfonylisocyanat durch Umsetzung von Chlorcyan und Schwefeltrioxid im Molverhältnis 1 : 1 bei Temperaturen von 100 bis 200°C sowie eine Anlage zur Durchführung des erfindungsgemäßen Verfahrens.

Aus der DE-PS 928 896 ist es bekannt, Stickstoff enthaltende chlorierte Schwefelverbindungen durch Umsetzung von Schwefeltrioxid mit Chlorcyan herzustellen. Setzt man dabei die genannten Einsatzstoffe im Molverhältnis 1 : 1 bei Temperaturen zwischen 100 und 200°C um, so erhält man N-Carbonylsulfamidsäurechlorid der Formel OCN SO$_2$ Cl, dem auch die Nomenklatur Chlorsulfonylisocyanat (CSI) zukommt.

Nachteilig ist bei der beschriebenen Herstellungsweise, daß sie zur kontinuierlichen Durchführung des Einsatzes von schlecht handhabbarem flüssigem Schwefeltrioxid bedarf.

Es ist daher Aufgabe der vorliegenden Erfindung, ein Verfahren zur kontinuierlichen Herstellung von Chlorsulfonylisocyanat aus Chlorcyan und Schwefeltrioxid sowie eine Anlage zu seiner Durchführung anzugeben, bei welchen die Umsetzung der Reaktanden in der Gasphase erfolgt. Das wird erfindungsgemäß dadurch erreicht, daß man zur Gewinnung des Chlorcyans in wäßrige Blausäure bei Temperaturen von 20 bis 70°C Chlorgas eindüst, die wäßrige Phase auskocht, das dabei gebildete gasförmige Gemisch aus Chlorcyan und Chlorwasserstoff nach Vereinigung mit dem direkt verdampften Chlorcyan einer Wasserwäsche unterwirft und schließlich das gasförmige Chlorcyan mehrstufig durch ein festes Trocknungsmittel strömen läßt : daß man in den Gasraum einer mit chlorsulfonylisocyanathaltiger Flüssigkeit mit Temperaturen von 90 bis 135°C teilweise gefüllten Reaktionszone gasförmiges Chlorcyan und Schwefeltrioxid einleitet und reagieren läßt ; daß man die aus der Reaktionszone abströmende Gasphase eine Fraktionierungszone passieren läßt ; daß man die oben aus der Fraktionierungszone austretende Dampfphase in einer nachgeschalteten ersten Kühlzone unter 100°C abgekühlt und dabei vollständig verflüssigt ; daß man die resultierende Flüssigkeit in eine Destillationszone einführt und fraktioniert destilliert und daß man die aus der Destillationszone abströmende Gasphase eine auf Temperaturen von 75 bis 100°C gehaltene zweite Kühlzone durchströmen läßt, wobei flüssiges Chlorsulfonylisocyanat anfällt, während die unter diesen Bedingungen nicht kondensierbaren Gase in die Reaktionszone zurückgeführt werden.

Das Verfahren gemäß der Erfindung kann weiterhin auch noch dadurch ausgestaltet sein, daß als Trocknungsmittel wasserfreies Calciumchlorid dient.

Eine Anlage zur Durchführung des erfindungsgemäßen Verfahrens kann gekennzeichnet sein durch einen Blausäure-Behälter, einen Chlorgas-Behälter, eine Chlorcyan-Reaktionskolonne, eine Waschkolonne, einen ersten Kühler, eine Auskochkolonne, einen zweiten Kühler und eine Destillationsblase, wobei der Blausäure-Behälter mit dem unteren Bereich der Chlorcyan-Reaktionskolonne verbunden ist und wobei eine mit dem Chlorgas-Behälter verbundene Zuführungsleitung von unten her in die Chlorcyan-Reaktionskolonne einmündet und wobei die Chlorcyan-Reaktionskolonne mit der über ihr angeordneten Waschkolonne strömungsmäßig verbunden ist und wobei eine Steigleitung den unteren Bereich der Chlorcyan-Reaktionskolonne mit der Auskochkolonne verbindet und wobei der zweite Kühler auf die Auskochkolonne aufgesetzt ist und wobei der obere Bereich des zweiten Kühlers mit dem unteren Bereich der Waschkolonne verbunden ist und wobei die Destillationsblase unterhalb der Auskochkolonne angeordnet ist ; mindestens einen Trockenmittel-Behälter, einen CSI-Generator, eine Roh-CSI-Kolonne, einen Fraktionsteiler und einen Roh-CSI-Kühler, wobei der Trockenmittel-Behälter einerseits über eine zweite Leitung mit dem oberen Bereich des ersten Kühlers und andererseits über eine dritte Leitung mit dem CSI-Generator verbunden ist und wobei auf den CSI-Generator die Roh-CSI-Kolonne, der Fraktionsteiler und der Roh-CSI-Kühler aufgesetzt sind ; einen Schwefeltrioxid-Behäler und einen Verdampfer, wobei der Schwefeltrioxid-Behälter und der Verdampfer durch eine vierte Leitung miteinander verbunden sind und wobei der Verdampfer und der CSI-Generator strömungsmäßig miteinander verbunden sind ; eine Rein-CSI-Destillationsblase, eine Rein-CSI-Kolonne, einen Rein-CSI-Fraktionsteiler und einen Rein-CSI-Kühler, wobei auf die zweite Destillationsblase die Rein-CSI-Kolonne, der Rein-CSI-Fraktionsteiler und der Rein-CSI-Kühler aufgesetzt sind und wobei der obere Bereich des Rein-CSI-Kühlers über eine sechste Leitung mit dem CSI-Generator verbunden ist und wobei eine fünfte Leitung vom Fraktionsteiler zur Rein-CSI-Destillationsblase führt und wobei vom Rein-CSI-Fraktionsteiler eine Produktleitung abgeht.

Diese Anlage kann auch noch dadurch weitergebildet sein, daß

a) die Zuführungsleitung an ihrem innerhalb der Chlorcyan-Reaktionskolonne befindlichen Ende eine Fritte trägt ;

b) die Waschkolonne von einem ersten Wasserzuführungsrohr durchdrungen ist ;

c) in den ersten Kühler von oben her ein zweites Wasserzuführungsrohr hineinragt ;

d) bis zu sechs, vorzugsweise bis zu drei Trockenmittel-Behälter strömungsmäßig hintereinander angeordnet sind ;

e) der CSI-Generator an seinem unteren Ende eine Ausstülpung aufweist, in welcher eine von einem Wärmeübertragungsmittel durchströmte Heizschlange angeordnet ist :

f) die Rein-CSI-Destillationsblase von einem Heizmantel umgeben ist.

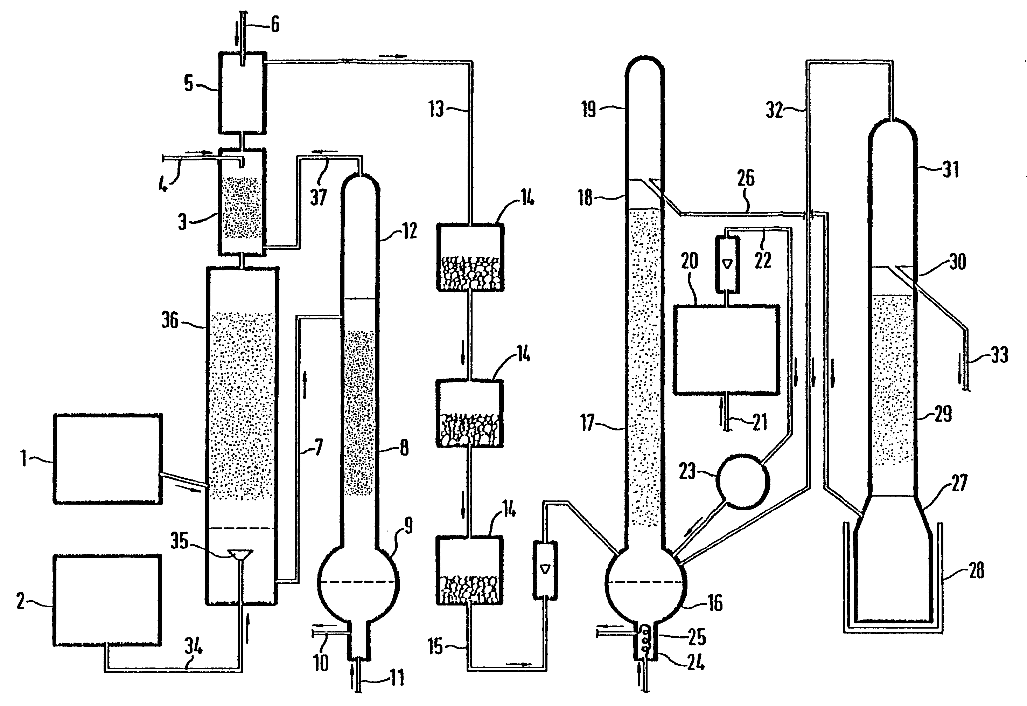

In der beigefügten Zeichnung ist eine Anlage zur Durchführung des erfindungsgemäßen Verfahrens schematisch dargestellt.

Aus einem verdünnte Blausäure enthaltenden Behälter 1 wird Blausäure in den unteren Bereich der Chlorcyan-Reaktionskolonne 36 dosiert, in welche über eine in einer Fritte 35 endenden Zuführungsleitung 34 von unten der Chlorgas aus einem Behälter 2 dosiert wird. Die Chlorcyan-Reaktionskolonne 36 ist strömungsmäßig mit einer über ihr angeordneten, Füllkörper enthaltenden Waschkolonne 3 verbunden, deren Füllkorper von oben her über ein erstes Wasserzuführungsrohr 4 beregnet werden. Oberhalb der Waschkolonne 3 und strömungsmäßig mit ihr verbunden befindet sich ein erster Kühler 5, in welchen oben ein zweites Wasserzuführungsrohr 6 einmündet. Während die Hauptmenge Chlorcyan über den Kopf der Waschkolonne 3 und den ersten Kühler 5 abströmt, wird chlorcyanhaltiges Wasser aus dem unteren Teil der Chlorcyan-Reaktionskolonne 36 über eine Steigleitung 7 in den oberen Teil einer Auskochkolonne 8 geführt, deren unteres Ende mit einer Destillationsblase 9 verbunden ist. Die in der Destillationsblase 9 befindliche wäßrige Phase ist über eine Ablaufleitung 10 und eine Zulaufleitung 11 mit einem nicht dargestellen Wärmeaustauscher verbunden ; aus dem Wärmeaustauscher-Kreislauf kann wäßrige Salzsäure kontinuierlich ausgeschleust werden. Auf der Auskochkolonne 8 befindet sich ein zweiter Kühler 12. Vom Kopf des zweiten Kühlers 12 strömt das ausgetriebene Chlorcyan über eine erste Leitung 37 in den unteren Bereich der Waschkolonne 3. Vom oberen Bereich des ersten Kühlers 5 geht eine zweite Leitung 13 ab, welche mit mehreren strömungsmäßig hintereinander geschalteten Trockenmittel-Behälteren 14 verbunden ist. Vom letzten Trockenmittel-Behälter 14 führt eine mit einer Dosiereinrichtung versehene, von reinem, trockenem Chlorcyan durchströmte dritte Leitung 15 zu einem CSI-Generator 16.

Der CSI-Generator 16 ist mit einer Ausstülpung 24 versehen, in welcher eine von einem Wärmeübertragungsmittel durchströmte Heizschlange 25 angeordnet ist. Auf den CSI-Generator 16 ist eine Roh-CSI-Kolonne 17 aufgesetzt, welche an ihrem oberen Ende einen Fraktionsteiler 18 und darauf einen Roh-CSI-Kühler 19 trägt.

Von einem Schwefeltrioxid-Behälter 20, welcher mit einer Preßluft-Leitung 21 verbunden ist, geht eine mit einer Dosiervorrichtung versehene vierte Leitung 22 zu einem Verdampfer 23 ab. Der Verdampfer 23 ist strömungsmäßig mit dem CSI-Generator 16 verbunden. Das aus dem Fraktionsteiler 18 austretende Kondensat läuft über eine fünfte Leitung 26 in eine Rein-CSI-Destillationsblase 27, welche von einem Heizmantel 28 umgeben ist. Auf die Rein-CSI-Destillationsblase 27 ist eine Rein-CSI-Kolonne 29 aufgesetzt, welche einen Rein-CSI-Fraktionsteiler 30 und darauf einen Rein-CSI-Kühler 31 trägt. Die im Rein-CSI-Kühler 31 nicht kondensierbaren Anteile werden über eine sechste Leitung 32 in den Gasraum des CSI-Generators 16 zurückgeführt.

Der Rein-CSI-Fraktionsteiler 30 ist mit einer Produktleitung 33 verbunden, aus welcher reines Chlorsulfonylisocyanat (CSI) abläuft.

Beispiel

In den unteren Bereich der Chlorcyan-Reaktionskolonne 36 (vergl. die Figur) wurden 22 l/h wäßrige Blausäure (20 Gew.-% HCN) eingeleitet, während aus der Fritte 35 4000 l/h Chlorgas austraten. Aus dem ersten Wasserzuführungsrohr 4 traten 120 l/h Beregnungswasser für die Waschkolonne 3 aus.

Das zu einem Teil im Wasser gelöste Chlorcyan wurde über die Steigleitung 7 in den oberen Teil der Auskochkolonne 8 geführt. Die in der Destillationsblase 9 enthaltene wäßrige Phase wurde durch Wärmeaustausch auf 104°C gehalten. Ein gasförmiges Chlorcyan-Chlorwasserstoff-Gemisch gelangte über die Auskochkolonne 8 und den zwiten Kühler 12 in die Waschkolonne 3, in welcher im wesentlichen nur Chlorwasserstoff ausgewaschen wurde. Das aus dem ersten Kühler 5 austretende gasförmige Chlorcyan strömte über eine zweite Leitung 13 drei hintereinandergeschalteten, mit wasserfreiem Calciumchlorid gefüllten Behältern 14 zu.

In dem CSI-Generator 16 befanden sich etwa 30 l einer weitgehend aus Chlorsulfonylisocyanat bestehenden Flüssigkeit, welche durch die von Wärmeöl durchströmte Heizschlange 25 auf einer Temperatur von 120 bis 130°C gehalten wurde. In den Gasraum des CSI-Generators 16 wurden über die dritte Leitung 15 5000 l/h Chlorcyan und aus dem Verdampfer 23 Schwefeltrioxid eingeleitet, wobei der Verdampfer 23 mit 5 l/h flüssigem, stabilisiertem Schwefeltrioxids aus dem Heizschrank 20 über die vierte Leitung 22 beaufschlagt wurde. Am Kopf der Roh-CSI-Kolonne 17 betrug die Temperatur der Gasphase zwischen 98 und 110°C ; die Gasphase wurde im Roh-CSI-Kühler 19 kondensiert. Das Kondensat gelangte über den Fraktionsteiler 18 und die fünfte Leitung 26 in die Rein-CSI-Destillationsblase 27.

Die Rein-CSI-Destillationsblase 27 wurde mit Hilfe des sie umgebenden Heizmantels 28 so erwärmt, daß die in ihr enthaltene flüssige Phase eine Temperatur von 121 bis 125°C aufwies. Die Temperatur des Dampfphase am Kopf der Rein-CSI-Kolonne 29 betrug 106 bis 107°C ; nach Kondensation dieser Dampfphase im Rein-CSI-Kühler 31 lief aus der Produktleitung 33 ein Chlorsulfonylisocyanat (CSI) mit einer Dichte $d_4^{20} = 1,626$ g/cm$^3$ ab.

Aus Chlorsulfonylisocyanat und Olefinen sind am Stickstoff nicht substituierte ß-Lactame erhältlich, aus denen der Aufbau hochpolymerer, der Naturseide ähnlicher Poly-ß-amide möglich ist.

Weiterhin entstehen aus Chlorsulfonylisocyanat mit Aldehyden, Ketonen, ß-Oxocarbonsäureestern und Benzylvinyläthern über eine gemeinsame Zwischenstufe Oxathiazinondioxide, die, wenn sie in C-5 und C-6 mit niedrigen Alkylresten substituiert sind, Süßstoffe mit einer auf eine 4 %ige wäßrige Rohrzuckerlöfsung bezogenen relativen Süße von mehr als 100 liefern.

**Ansprüche**

1. Verfahren zur kontinuierlichen Herstellung von Chlorsulfonylisocyanat durch Umsetzung von Chlorcyan und Schwefeltrioxid im Molverhältnis 1 : 1 bei Temperaturen von 100 bis 200°C, dadurch gekennzeichnet, daß man zur Gewinnung des Chlorcyans in wäßrige Blausäure bei Temperaturen von 20 bis 70°C Chlorgas eindüst, die wäßrige Phase auskocht, das dabei gebildete gasförmige Gemisch aus Chlorcyan und Chlorwasserstoff nach Vereinigung mit dem direkt verdampften Chlorcyan einer Wasserwäsche unterwirft und schließlich das gasförmige Chlorcyan mehrstufig durch ein festes Trocknungsmittel strömen läßt ; daß man in den Gasraum einer mit chlorsulfonylisocyanathaltiger Flüssigkeit mit Temperaturen von 90 bis 135°C teilweise gefüllten Reaktionszone gasförmiges Chlorcyan und Schwefeltrioxid einleitet und reagieren läßt ; daß man die aus der Reaktionszone abströmende Gasphase eine Fraktionierungszone passieren läßt ; daß man die oben aus der Fraktionierungszone austretende Dampfphase in einer nachgeschalteten ersten Kühlzone unter 100°C abgekühlt und dabei vollständig verflüssigt ; daß man die resultierende Flüssigkeit in eine Destillationszone einführt und fraktioniert destilliert und daß man die aus der Destillationszone abströmende Gasphase eine auf Temperaturen von 75 bis 100°C gehaltene zweite Kühlzone durchströmen läßt, wobei flüssiges Chlorsulfonylisocyanat anfällt, während die unter diesen Bedigungen nicht kondensierbaren Gase in die Reaktionszone zurückgeführt werden.

2. Verfahren nach Anspruch 1, dadurch gekennzeichnet, daß als Trocknungsmittel wasserfreies Calciumchlorid dient.

3. Anlage zur Durchführung des Verfahrens nach den Ansprüchen 1 oder 2, gekennzeichnet durch einen Blausäure-Behälter (1), einen Chlorgas-Behälter (2), eine Chlorcyan-Reaktionskolonne (36), eine Waschkolonne (3), einen ersten Kühler (5), eine Auskochkolonne (8), einen zweiten Kühler (12) und eine Destillationsblase (9), wobei der Blausäure-Behälter (1) mit dem unteren Bereich der Chlorcyan-Reaktionskolonne (36) verbunden ist und wobei eine mit dem Chlorgas-Behälter (2) verbundene Zuführungsleitung (34) von unten her in die Chlorcyan-Reaktionskolonne (36) einmündet und wobei die Chlorcyan-Reaktionskolonne (36) mit der über ihr angeordneten Waschkolonne (3) strömungsmäßig verbunden ist und wobei eine Steigleitung (7) den unteren Bereich der Chlorcyan-Reaktionskolonne (36) mit der Auskochkolonne (8) verbindet und wobei der zweite Kühler (12) auf die Auskochkolonne (8) aufgesetzt ist und wobei der obere Bereich des zweiten Kühlers (12) mit dem unteren Bereich der Waschkolonne (3) verbunden ist und wobei die Destillationsblase (9) unterhalb der Auskochkolonne (8) angeordnet ist ; mindestens einen Trockenmittel-Behälter (14), einen CSI-Generator (16), eine Roh-CSI-Kolonne (17), einen Fraktionsteiler (18) und einen Roh-CSI-Kühler (19), wobei der Trockenmittel-Behälter (14) einerseits über eine zweite Leitung (13) mit dem oberen Bereich des ersten Kühlers (5) und andererseits über eine dritte Leitung (15) mit dem CSI-Generator (16) verbunden ist und wobei auf den CSI-Generator (16) die Roh-CSI-Kolonne (17), der Fraktionsteiler (18) und der Roh-CSI-Kühler (19) aufgesetzt sind ; einen Schwefeltrioxid-Behälter (20) und einen Verdampfer (23), wobei der Schwefeltrioxid-Behälter (20) und der Verdampfer (23) durch eine vierte Leitung (22) miteinander verbunden sind und wobei der Verdampfer (23) und der CSI-Generator (16) strömungsmäßig miteinander verbunden sind ; eine Rein-CSI-Destillationsblase (27), eine Rein-CSI-Kolonne (29), einen Rein-CSI-Fraktionsteiler (30) und einen Rein-CSI-Kühler (31), wobei auf die zweite Destillationsblase (27) die Rein-CSI-Kolonne (29), der Rein-CSI-Fraktionsteiler (30) und der Rein-CSI-Kühler (31) aufgesetzt sind und wobei der obere Bereich des Rein-CSI-Kühlers (31) über eine sechste Leitung (32) mit dem CSI-Generator (16) verbunden ist und wobei eine fünfte Leitung (26) vom Fraktionsteiler (18) zur Rein-CSI-Destillationsblase (27) führt und wobei vom Rein-CSI-Fraktionsteiler (30) eine Produktleitung (33) abgeht.

4. Anlage nach Anspruch 3, dadurch gekennzeichnet, daß die Zuführungsleitung (34) an ihrem innerhalb der Chlorcyan-Reaktionskolonne (36) befindlichen Ende eine Fritte (35) trägt.

5. Anlage nach Anspruch 3 oder 4, dadurch gekennzeichnet, daß die Waschkolonne (3) vom einem ersten Wasserzuführungsrohr (4) durchdrungen ist.

6. Anlage nach mindestens einem der Ansprüche 3 bis 5, dadurch gekennzeichnet, daß in den ersten Kühler (5) von oben her ein zweites Wasserzuführungsrohr (6) hineinragt.

7. Anlage nach mindestens einem der Ansprüche 3 bis 6, dadurch gekennzeichnet, daß bis zu sechs, vorzugsweise bis zu drei Trockenmittel-Behälter (14) strömungsmäßig hintereinander angeordnet sind.

8. Anlage nach mindestens einem der Ansprüche 3 bis 7, dadurch gekennzeichnet, daß der CSI-Generator (16) an seinem unteren Ende eine Ausstülpung (24) aufweist, in welcher eine von einem Wärmeübertragungsmittel durchströmte Heizschlange (25) angeordnet ist.

9. Anlage nach mindestens einem der Ansprüche 3 bis 8, dadurch gekennzeichnet, daß die Rein-CSI-Destillationsblase (27), von einem Heizmantel (28) umgeben ist.


## Claims

1. A process for the continuous preparation of chlorosulfonyl isocyanate by reacting cyanogen chloride and sulfur trioxide in the molar ratio of 1 : 1 at temperatures from 100 to 200°C, which comprises obtaining the cyanogen chloride by injecting chlorine gas into aqueous hydrogen cyanide at temperatures from 20 to 70°C, boiling off the aqueous phase, subjecting the gaseous mixture thus formed of cyanogen chloride and hydrogen chloride after combining it with the directly vaporized cyanogen chloride to a water wash and finally allowing the gaseous cyanogen chloride to flow through a solid drying agent in a multistage arrangement, introducing gaseous cyanogen chloride and sulfur trioxide into the gas space of a reaction zone partly filled with a liquid containing chlorosulfonyl isocyanate at temperatures from 90 to 135°C, and allowing them to react, passing the gas phase flowing out of the reaction zone into a fractionating zone, cooling the vapor phase escaping from the top of the fractionating zone to below 100°C downstream in a first cooling zone and thus completely liquefying it, introducing the resulting liquid into a distillation zone and subjecting it to a fractional distillation, and allowing the gas phase flowing off out of the distillation zone to flow through a second cooling zone which is maintained at temperatures from 75 to 100°C, liquid chlorosulfonyl isocyanate being obtained, while the gases which cannot be condensed under these conditions are fed back into the reaction zone.

2. The process as claimed in claim 1, wherein anhydrous calcium chloride serves as drying agent.

3. A plant for carrying out the process as claimed in claims 1 or 2, comprising a hydrogen cyanide container (1), a chlorine gas container (2), a cyanogen chloride reaction column (36), a wash column (3), a first condenser (5), a boiling-off column (8), a second condenser (12), and a distillation boiler (9), where the hydrogen cyanide container (1) is connected to the lower region of the cyanogen chloride reaction column (36) and where a feed line (34) connected to the chlorine gas container (2) leads into the cyanogen chloride reaction column (36) from below and where the cyanogen chloride reaction column (36) is flow connected to the wash column (3) arranged above it and where a riser (7) connects the lower region of the cyanogen chloride reaction column (36) to the boiling-off column (8) and where the second condenser (12) is mounted on the boiling-off column (8) and where the upper region of the second condenser (12) is connected to the lower region of the wash column (3) and where the distillation boiler (9) is arranged below the boiling-off column (8) ; at least one drying agent container (14), a CSI generator (16), a crude CSI column (17), a fractionator (18) and a crude CSI condenser (19), where the drying agent container (14) is connected, on the one hand, via a second line (13) to the upper region of the first condenser (5) and, on the other hand, via a third line (15) to the CSI generator (16) and where the crude CSI column (17), the fractionator (18) and the crude CSI condenser (19) are mounted on the CSI generator (16) ; a sulfur trioxide container (20) and a vaporizer (23), where the sulfur trioxide container (20) and the vaporizer (23) are connected to each other by a fourth line (22) and where the vaporizer (23) and the CSI generator (16) are flow connected to each other ; a pure CSI distillation boiler (27), a pure CSI column (29), a pure CSI fractionator (30) and a pure CSI condenser (31), where the pure CSI column (29), the pure CSI fractionator (30) and the pure CSI condenser (31) are mounted on the second distillation boiler (27) and where the upper region of the pure CSI condenser (31) is connected via a sixth line (32) to the CSI generator (16) and where a fifth line (26) leads from the fractionator (18) to the pure CSI distillation boiler (27) and where a product line (33) leads off from the pure CSI fractionator (30).

4. The plant as claimed in claim 3, wherein the feed line (34) has a frit (35) on its end which is situated inside the cyanogen chloride reaction column (36).

5. The plant as claimed in claim 3 or 4, wherein a first water feed pipe (4) leads into the wash column (3).

6. The plant as claimed in at least one of claims 3 to 5, wherein a second water feed pipe (6) projects into the first condenser (5) from above.

7. The plant as claimed in at least one of claims 3 to 6, wherein up to six, preferably up to three, drying agent containers (14) are consecutively flow arranged.

8. The plant as claimed in at least one of claims 3 to 7, wherein the CSI generator (16) has an outward bulge (24) at its lower end, in which a heating coil (25) is arranged through which a heat transfer medium flows.

9. The plant as claimed in at least one of claims 3 to 8, wherein the pure CSI distillation boiler (27) is surrounded by a heating jacket (28).


## Revendications

1. Un procédé pour la fabrication continue d'isocyanate de chlorosulfonyle à partir de chlorure de cyanogène et de trioxyde de soufre, dans le rapport molaire 1 : 1 à des températures de 100 à 200°C, caractérisé en ce que l'on injecte du chlore gazeux dans l'acide cyanhydrique aqueux à des températures de 20 à 70°C pour l'obtention du chlorure de cyanogène, on fait bouillir la phase aqueuse, on soumet le mélange gazeux de chlorure de cyanogène et de chlorure d'hydrogène ainsi formé, après combinaison avec le chlorure de cyanogène directement évaporé, à un lavage à l'eau et enfin on fait passer le chlorure de cyanogène gazeux en plusieurs étapes à travers un agent desséchant solide ; en ce que l'on envoie et on fait réagir le chlorure de cyanogène gazeux et le trioxyde de soufre dans l'atmosphère d'une zone de réaction partiellement remplie d'un liquide contenant de l'isocyanate de chlorosulfonyle à une température de 90 à 135°C ; en ce que l'on fait passer la phase gazeuse s'écoulant de la zone de réaction à travers une zone de fractionnement ; en ce que l'on refroidit au-dessous de 100°C dans une première zone de refroidissement branchée en aval la phase vapeur sortant par le haut de la zone de fractionnement et on la liquéfie ainsi complètement ; en ce que l'on envoie le liquide résultant dans une zone de distillation où on le soumet à la distillation fractionnée ; et en ce que l'on fait passer la phase gazeuse s'écoulant de la zone de distillation à travers une seconde zone de refroidissement maintenue à des températures de 75 à 100°C, en obtenant l'isocyanate de chlorosulfonyle liquide, tandis que les gaz non condensables dans ces conditions sont recyclés dans la zone de réaction.

2. Procédé selon la revendication 1, caractérisé en ce que l'on utilise du chlorure de calcium anhydre comme agent desséchant.

3. Installation pour la mise en oeuvre du procédé selon la revendication 1 ou 2, caractérisée par un réservoir d'acide cyanhydrique (1), un réservoir de chlore gazeux (2), une colonne de réaction en chlorure de cyanogène (36), un colonne de lavage (3), un premier réfrigérant (5), une colonne d'ébullition (8), un second réfrigérant (12) et un ballon à distiller (9), de sorte que le réservoir d'acide cyanhydrique (1) est relié à la partie inférieure de la colonne de réaction en chlorure de cyanogène (36) et de sorte qu'une conduite d'amenée (34) reliée au réservoir de chlore gazeux (2) débouche par le bas dans la colonne de réaction en chlorure de cyanogène (36) et en ce que la colonne de réaction en chlorure de cyanogène (36) est reliée dans le sens de l'écoulement avec la colonne de lavage (3) disposée au-dessus d'elle et de sorte qu'une conduite ascendante (7) relie la partie inférieure de la colonne de réaction en chlorure de cyanogène (36) avec la colonne d'ébullition (8) et de sorte que le second réfrigérant (12) est disposé sur la colonne d'ébullition (8) et de sorte que la partie supérieure du second réfrigérant (12) est reliée à la partie inférieure de la colonne de lavage (3) et de sorte que le ballon à distiller (9) est disposé au-dessous de la colonne d'ébullition (8) ; par au moins un récipient d'agent desséchant (14), un générateur de CSI (16), une colonne de CSI brut (17), un séparateur de fraction (18) et un réfrigérant de CSI brut (19), de sorte que le récipient d'agent desséchant (14) est relié, d'une part, à la partie supérieure du premier réfrigérant (5) par une seconde conduite (13) et, d'autre part, au générateur de CSI (16) par une troisième conduite (15) et de sorte que la colonne de CSI brut (17), le séparateur de fraction (18) et le réfrigérant de CSI brut (19) sont disposés sur le générateur de CSI (16) ; par un récipient de trioxyde de soufre (20) et un évaporateur (23), de sorte que le récipient de trioxyde de soufre (20) et l'évaporateur (23) sont reliés entre eux par une quatrième conduite (22) et de sorte que l'évaporateur (23) et le générateur de CSI (16) sont reliés entre eux dans le sens de l'écoulement ; par un ballon de distillation du CSI pur (27), une colonne de CSI pur (29), un séparateur de fraction de CSI pur (30) et un réfrigérant de CSI pur (31), de sorte que la colonne de CSI pur (29), le séparateur de fraction de CSI pur (30) et le réfrigérant de CSI pur (31) sont disposés sur le second ballon de distillation (27) et de sorte que la partie supérieure du réfrigérant de CSI pur (31) est reliée par une sixième conduite (32) au générateur de CSI (16) et de sorte qu'une cinquième conduite (26) relie le séparateur de fraction (18) au ballon de distillation de CSI pur (27) et de sorte qu'une conduite de produit (33) part du séparateur de fraction de CSI pur (30).

4. Installation selon la revendication 3, caractérisée en ce que la conduite d'amenée (34) porte une plaque de verre fritté (35) à son extrémité à l'intérieur de la colonne de réaction en chlorure de cyanogène (36).

5. Installation selon la revendication 3 ou 4, caractérisée en ce que la colonne de lavage (3) est traversée par un premier tube d'amenée d'eau (4).

6. Installation selon l'une au moins des revendications 3 à 5, caractérisée en ce qu'un deuxième tube d'amenée d'eau (6) pénètre par le haut dans le premier réfrigérant (5).

7. Installation selon l'une au moins des revendications 3 à 6, caractérisée en ce qu'un à six récipients d'agent desséchant (14), de préférence jusqu'à trois, sont disposés l'un après l'autre dans le sens de l'écoulement.

8. Installation selon l'une au moins des revendications 3 à 7, caractérisée en ce que le générateur de CSI (16) comporte à son extrémité inférieure une excroissance (24) dans laquelle est disposé un serpentin chauffant (25) parcouru par un agent caloporteur.

9. Installation selon l'une au moins des revendications 3 à 8, caractérisée en ce que le ballon de distillation du CSI pur (27) est entouré par une enveloppe chauffante (28).